Europäisches Patentamt

European Patent Office

Office européen des brevets

(19)

(11) Veröffentlichungsnummer: **0 167 825**
**B1**

(12) **EUROPÄISCHE PATENTSCHRIFT**

(45) Veröffentlichungstag der Patentschrift: **08.08.90**

(21) Anmeldenummer: **85106926.0**

(22) Anmeldetag: **04.06.85**

(51) Int. Cl.⁵: **A 61 K 9/50, A 61 K 9/10**

(54) Lipidnanopellets als Trägersystem für Arzneimittel zur peroralen Anwendung.

(30) Priorität: **08.06.84 DE 3421468**

(43) Veröffentlichungstag der Anmeldung:
**15.01.86 Patentblatt 86/03**

(45) Bekanntmachung des Hinweises auf die
Patenterteilung:
**08.08.90 Patentblatt 90/32**

(84) Benannte Vertragsstaaten:
**AT BE CH DE FR GB IT LI LU NL SE**

(56) Entgegenhaltungen:
EP-A-0 032 578
EP-A-0 042 249
EP-A-0 045 008
EP-A-0 152 379
WO-A-83/00294
FR-A-2 515 960

**Die Akte enthält technische Angaben, die nach
dem Eingang der Anmeldung eingereicht
wurden und die nicht in dieser Patentschrift
enthalten sind.**

(73) Patentinhaber: **Dr. Rentschler Arzneimittel
GmbH & Co.**
**Postfach 320 Mittelstrasse 18**
**D-7958 Laupheim (DE)**

(72) Erfinder: **Speiser, Peter, Prof. Dr.**
**Clausiusstrasse 25**
**CH-8042 Zürich (CH)**

(74) Vertreter: **Dipl.-Ing. Schwabe, Dr. Dr. Sandmair,
Dr. Marx**
**Stuntzstrasse 16**
**D-8000 München 80 (DE)**

Courier Press, Leamington Spa, England.

**Beschreibung**

Gegenstand der Erfindung ist ein untenstehend näher definiertes arzneimittelhaltiges Trägersystem, das aus Lipidnanopellets besteht die eine durchschnittliche Teilchengröße von 50—1 000 nm, vorzugsweise 80—800 nm aufweisen und bei Raumtemperatur einen festen Aggregatzustand besitzen. Das Trägersystem ist zur peroralen Verabreichung geeignet.

Arzneiformen für biologisch aktive Stoffe zur gezielten Anwendung an bestimmten Stellen im Organismus und zur Vermeidung einer schnellen Ausscheidung sind Formulierungen, bei denen ein Wirkstoff an bestimmte Trägersubstanzen gebunden oder darin eingeschlossen ist, so daß keine vorzeitige Freigabe erfolgt. Nach peroraler Verabreichung erfolgt die Freisetzung der Wirkstoffe dann in der Regel im Gastrointestinaltrakt, wobei die Arzneiform entweder in Partikel zerfällt und der Wirkstoff durch die Verdauungsflüssigkeit in Lösung gebracht wird, oder aber der Wirkstoff aus der intakten Darreichungsform durch Diffusion herausgelöst wird. Dieser Vorgang kann schnell oder zeitlich verlangsamt (retardiert) vonstatten gehen. In allen Fällen passiert der Wirkstoff nach der anschließenden Resorption die Leber ("first pass Effekt"), wo er teilweise oder vollständig metabolisiert, d.h. chemisch umgewandelt wird und somit nur zum Teil als Wirksubstanz den Wirkort erreicht.

Insbesondere zur retardierten Freigabe von Wirkstoffen wurden seit langem kleine Partikel entwickelt, in denen der Wirkstoff eingeschlossen ist. Die zur Herstellung derartiger Partikel notwendigen Hilfsstoffe sind jedoch häufig physiologisch nicht akzeptabel oder die Herstellverfahren sind aufwendig, oder die Stabilität der Partikel ist nur gering. Peroral verabreicht, wird der Wirkstoff entweder durch Diffusion im Verdauungstrakt aus dem Partikel herausgelöst, oder durch enzymatischen Abbau der Partikelhülle freigesetzt. Der resorbierte Wirkstoff unterliegt in beiden Fällen dann jedoch wiederum dem "first pass Effekt" in der Leber.

Zu derartigen kleinen Partikeln gehören Mikrokapseln, Nanokapseln und Liposome.

Die WO—A—8300294 betrifft ein Arzneimittelträgersystem aus Micellen, die aus einer Gallensäure, einer Fettsäure und einem Monoglycerid aufgebaut sind.

Die EP—A—32 578 beschreibt Bilayer-Vesikel die als Trägersystem für biologisch und/oder pharmazeutische Präparate verwendbar sind. Die Vesikel werden aus einer im wässrigen Medium Bilayer bildenden Substanz, z.B. Phospolipid, und einem Detergens, z.B. Cholat, in Form vom Mizellen in kolloidaler Lösung gebildet.

Aus der EP—A—152 379 ist ein Verfahren zur Herstellung einer pharmazeutischen Zusammensetzung bekannt, die unilamellare Liposomen enthält, die aus einer amphipatischen Verbindung mit biologischer Wirkung und einem Phospholipid bestehen. Die Liposomen können als Arzeimittelträger verwendet werden.

Die EP—A—102 324 betrifft ein Verfahren zur Herstellung vom unilamellaren Liposomen in wässriger Phase bei dem man eine homogene Mischung eines ionischen Tensids und eines Lipids dispergiert. Die Liposomen können als Wirkstoffträger verwendet werden.

Mikrokapseln aus Gelatine oder Cellulosderivaten sind allgemein durch den Nachteil gekennzeichnet, daß zu ihrer Herstellung ein aufwendiges Verfahren erforderlich ist und daß ihre Partikelgröße im Mikrometerbereich liegt.

Nanokapseln werden im allgemeinen auf der Basis von Polyacrylamiden sowie Polycyanoacrylaten und anderen synthetischen Ausgangsstoffen hergestellt. Diese sind mit dem Nachteil behaftet, daß sie eine gewisse Toxizität aufweisen und daher häufig nicht zur Anwendung am Menschen geeignet sind.

Liposome sind hochgeordnete Gebilde auf der Basis von Phospholipiden aus ein oder mehreren Lipiddoppelschichten, die eine Membran bilden, in die räumlich in die Zwischenräume passende Stoffe eingeschlossen werden. Ihre Größe hängt davon ab, ob es sich um multilaminare oder kleinere monolaminare Liposome handelt. Die Liposome haben den Nachteil, daß sie wenig stabil sind.

Volkheimer (Adv. in Pharmacol. Chemother. 14 (1977), S. 163—187) hat anhand von Stärkekörnern nachgewiesen, daß kleine feste Partikel aus dem Darm unverändert im Blut und Urin wiederzufinden sind. Der als "Persorption" bezeichnete Vorgang des Transportes von intakten Partikeln durch die Darmwand ist jedoch nur sehr unvollständig. Volkheimer nimmt an, daß nur 1 von 50 000 persorbierbaren Partikeln tatsächlich persorbiert wird. Dies ist im Falle von Stärke auch nicht überraschend, da Stärkekörner Partikeldurchmesser von z.B. 2—10 µm für Reisstärke und 10—25 µm für Maisstärke aufweisen. Stärke weist zudem starke hydrophile Eigenschaften auf, während im Darm bevorzugt lipophile Substanzen absorbiert werden.

Es ist weiterhin bekannt, daß langkettige Fettsäuren mit Kettenlängen von mehr als 12 Kohlenstoffatomen nicht der Leber, sondern bevorzugt dem Lymphsystem zugeführt werden, und daß durch einen speziellen Persorptionsvorgang, die Endozytose, kleine Tröpfchen oder Feststoffpartikel die Darmwand passieren können, die dann in den Lymphstrom abgegeben werden.

In der US—A—4.331.654 bzw. europäischen Patentanmeldung EP—A—0042249 werden zur intraarteriellen Injektion mangetisch lokalisierbare, biodegradierbare Lipidteilchen als Trägermaterialien für Wirkstoffe mit einer Teilchengröße von unter 5 000 nm, vorzugsweise 1 000—2 000 nm beschrieben, die ein oder mehrere oberflächenaktive Substanzen enthalten. Als Lipide dienen Fetsäuren mit Schmelzpunkten zwischen 30 und 100°C, insbesondere gesättigte Fettsäuren, höhermolekulare Alkohole, Mono-, Di- und Triglyceride einschließlich Glycerinester der Fettsäuren, Phospholipide, Sterole und Cerobroside. Die Lipidteilchen schmelzen oberhalb 30°C. Als oberflächenaktive Substanzen sind sowohl ionogene als nichtionogene genannt, wie Polyoxyethylensorbitanmonooleat, Salze langkettiger aliphatiischer Alkohole, quarernäre Ammoniumsalze und Lecithin. Als Wirkstoff wird das anorganische Magnetit, welches in der Lipidphase nicht löslich ist, verwendet, wobei die Teilchen dadurch erzeugt werden, daß oberhalb des Schmelzpunktes des Lipids in Wasser eine Dispersion erzeugt wird, aus der nach Abkühlen die festen Mikropartikeln durch Lyophilisierung isoliert werden.

Aufgabe der Erfindung ist es, mit Arzneistoffen beladbare Partikel herzustellen, die klein genug sind, um persorbiert zu werden, die ausreichend lipophil und physiologisch verträglich sind, um den in ihnen enthaltenen Arzneistoff weitgehend unverändert durch die Darmwand zu transportieren. Dadurch wird eine Verbesserung der Zurverfügungstellung am Wirkort (Resorptionsverbesserung) von solchen Wirkstoffen erreicht, die aus den bekannten peroral verabreichbaren Darreichungsformen schlecht resorbiert werden, da sie schlecht löslich sind, im Verdauungstrakt nicht oder nicht ausreichend resorbiert oder zu schnell bzw. in zu hohem Maße metabolisiert werden, oder bereits im Verdauungstrakt durch enzymatische oder chemische Einflüsse zerstört werden. Dabei sollen die Partikeln bei Raumtemperatur fest sein.

Erfindungsgemäß wird die Aufgabe durch ein Trägersystem gelöst, das aus einer ultrafeinen kolloidalen Suspension von Lipidnanopellets besteht, die bei Raumtemperatur einen festen Aggregatzustand besitzen, einen Teilchendurchmesser von 50—1 000 nm, insbesondere 80—800 nm aufweisen, in der Suspension in einer Konzentration von 1—20 Gew.% vorliegen, wobei die Lipidteilchen aus einem Gemisch von Lipiden mit grenzflächenaktiven Substanzen bestehen, deren Verhältnis in den Teilchen 1:0,01 bis 1:2,2 beträgt und die Teilchen 5—70 Gew.-% Lipide, 0,01—70 Gew.-% grenzflächenaktive Stoffe und 0,05—25 Gew.-% wirkstoff enthalten.

Die in das Trägersystem eingebrachten Wirkstoffe sind während der Herstellung der Lipidnanopellets in den Lipiden grundsätzlich gelöst, können jedoch auch kristallin oder amorph oder als Gemisch derartiger kristallographischer Zustände vorliegen, wenn nach Erkalten auf Raumtemperatur die Wirkstoffe auskristallisieren oder ausgefällt werden.

Das Einbringen der Wirkstoffe erfolgt direkt in das geschmolzene Lipid oder Lipidgemisch oder in ein Schmelzgemisch aus Lipid und grenzflächenaktivem Stoff oder kann durch Aufnehmen des grenzflächenaktiven Stoffes und Wirkstoffes in einem organischen flüchtigen Lösungsmittel wie chlorierten Kohlenwasserstoffen, z.B. Chloroform, Tetrachlorkohlenstoff, Methylenchlorid oder Alkoholen wie Ethanol und Einbringen dieser Lösung in das geschmolzene Lipid erfolgen. Nach sorgfältigem Einmischen bzw. Lösung unter Rühren oder Schütteln wird, sofern ein flüchtiges Lösungsmittel verwendet wird, dieses wieder abgedampft.

Verwendet werden Lipide wie Mono-, Di- und Triglyceride von gesättigten geradkettigen Fettsäuren mit 12—30 Kohlenstoffatomen wie Laurinsäure, Myristinsäure, Palmitinsäure, Stearinsäure, Arachinsäure, Behensäure, Lignocerinsäure, Cerotinsäure, Melissinsäure, sowie deren Ester, andere mehrwertiger Alkohole wie z.B. Ethylenglykol, Propylenglykol, Mannitol, Sorbitol, gesättigte Fettalkohole mit 12—22 Kohlenstoffatomen wie Laurylalkohol, Myristylalkohol, Cetylalkohol, Stearylalkohol, Arachidylalkohol, Behenylalkohol, gesättigte Wachsalkohole mit 24—30 Kohlenstofatomen wie Lignocerylalkohol, Cerylalkohol, Cerotylalkohol, Myricylalkohol.

Diese physiologisch akzeptable grenzflächenaktive Substanzen werden die physiologischen Gallensalze wie Natriumcholat, Natriumdehydrocholat, Natriumdeoxycholat, Natriumglykocholat, Natriumtaurocholat bevorzugt.

Tierische und pflanzliche Phospholipide wie Lecithine und ihre hydrierten Formen aber auch Polypeptide wie Gelatine mit ihren modifizierten Formen können ebenso verwendet werden.

Als synthetische grenzflächenaktive Substanzen eignen sich die Salze der Sulfobernsteinsäureester, Polyoxyethylensorbitanester, Sorbitanester und Sorbitanether, Polyoxyethylenfettalkoholether, Polyoxyethylenstearinsäureester sowie entsprechende Mischkondensate von Polyoxyethylen- mit Polyoxypropylenether, z.B. Pluronics®, ethoxylierte gesättigte Glyceride, z.B. Labrafile®, partielle Fettsäure-Glyceride und Polyglycide, z.B. Gelucire®.

Als Arzneiwirkstoff, mit denen das Trägersystem aus Lipid und grenzflächenaktivem Stoff beladbar ist, eignen sich insbesondere solche Wirkstoffe, die eine schlechte Bioverfügbarkeit aufweisen, d.h. schlecht löslich sind, im Verdauungstrakt nicht oder nicht ausreichend resorbiert oder zu schnell bzw. in zu hohem Maße metabolisiert werden, oder im Verdauungstrakt durch enzymatische oder chemische Einflüsse zerstört werden.

Besonders geeignete Wirkstoffe sind:

1. Insuline, wie
   natürliche, semisynthetische,
   synthetische Insuline
   Proinsulin

2. Antidiabetika, wie
   Glipizid
   Glicazid
   Ciglitazon

3. Vitamine, wie
   Vitamin A
   Vitamin B

4. Anticoagulantien, wie
   Heparin
   Gabexat-Mesilat

EP 0 167 825 B1

5.  Fibrinolytica, wie
        Urokinase
        Plasminogen Aktivator

6.  Antithrombotika, wie
        Suloctidil
        Nafazatrom
        Picotamid
        Heparin-Oligasaccharide
        Antithrombin III

7.  Lipidsenker, wie
        Beclobrat
        Bezafibrat
        Etofibrat
        Fenofibrat

8.  Blutfraktionen, wie
        Albumine
        Antithrombin
        Faktor IX
        Faktor VIII
        Haptoglobulin

9.  Herzglycoside, wie
        Digitoxin
        Digoxin
        Methyldigoxin
        Acetyldigoxin
        K-Strophantin

10. Vasodilatatoren, wie
        Molsidomin
        Hydralazin
        Dihydralazin
        Nicorandil

11. Calciumantagonisten, wie
        Diltiazem
        Flunarizin
        Gallopamil
        Verapamil
        Nifedipin
        Nicardipin
        Nimodipin
        Nitrendipin
        Lipoflazin
        Niludipin

12. ACE-Hemmer, wie
        Captopril
        Enalapril
        SA-446

13. Antihypertensiva, wie
        Minoxidil
        Dihydroergotoxin
        Dihydroergotoxin-Mesilat
        Endralazin

14. α+β-Blocker, wie
        Labetalol
        Sulfinalol
        Bucindolol

15. Diuretika, wie
        Triamteren
        Hydrochlorothiazid
        Furosemid
        Piretanid
        Metolazon

16. Peripherwirksame Vasodilatatoren, wie
        Buflomedil
        Minoxidil
        Cadralazin
        Propentofyllin

17. Antihypotensiva, wie
        Dihydroergotamin
        Dihydroergotamin-Mesilat
        Gepefrin

18. Beta-Blocker, wie
        Talinolol
        Propranolol
        Atenolol
        Metoprolol
        Nadolol
        Pindolol
        Oxprenolol
        Labetalol

19. Systemisch wirkende Antimykotica, wie
        Ketoconazol
        Griseofulvin

20. Contrazeptiva, wie
        Binovum
        Desogestrel
        Triquilar

21. Steroidhormone, wie
        Testosteron
        Testosteronundecanoat
        Progesteron
        Pregnenolon
        Corticosteron
        Cortisol
        Cortison
        Prednison
        Prednisolon
        Methylprednisolon
        Dexamethason

22. Prostaglandine, Prostacycline, wie
        Alprostadil
        Carboprost
        Epoprostenol
        Sulproston

23. Lactationshemmer, wie
        Bromocryptin
        Metergolin

24. Wachstumshormone, wie
        Somatotropin

25. Somatostatin, wie
    Stilamin
    Somatostatin und seine Derivate

26. Cephalosporine, wie
    Cefamandol
    Cefmenoxim
    Cefoperazon
    Ceftizoxim
    Cefalexin
    Cefalotin
    Cefazedon
    Cefmenoxim
    Cefotaxim
    Cefoxitin
    Cetsulodin

27. Antibiotica, wie
    Fosfomycin
    Fosmidomycin
    Rifapentin

28. Antiviralia, wie
    Aciclovir
    Metisoprenol
    Tromantadin
    Vidarabin
    Vidarabin-Na-Phosphat
    Immunglobuline

29. Interferone, Lymphokine, wie
    α-Interferon
    β-Interferon
    γ-Interferon

30. Vaccine, wie
    Corynebakterium parrum
    Hepatitis B Vaccine
    Lactobacillus Vaccine
    Pneumococcen Vaccine

31. Zytostatika, wie
    Chlormethin
    Cyclophosphamid
    Melphalan
    Chlorambucil
    Busulfan
    Thiotepa
    Methotrexat
    5-Fluoruracil
    Cytarabin
    6-Mercaptopurin
    6-Thioguanin
    Vincristin
    Vinblastin
    Vindesin
    Actinomycin D
    Mitomycin C
    Mitramycin
    Doxorubicin
    Bleomcyin
    Cisplatin
    Procarbacin
    Estramustin

32. Radiodiagnostika, wie
    Aminofostin
    Misonidazol

33. Antirheumatika, wie
    Indometacin
    Diclofenac
    Ibuprofen
    Ibuproxam
    Ketoprofen
    Pirprofen
    Suprofen

34. Antimigränemittel, wie
    Clonidin
    Flunarizin
    Metergolin
    Nadolol
    Dopaminantagonisten

35. Enkephaline, wie
    Metkephamid
    β-Endorphin
    Enkephalin

36. Antiparkinsonmittel, wie
    Lisuridhydrogenmaleat
    Memantin
    Piribedil
    Mesulergin
    Desocryptin

37. Vasodilatatoren, zerebralwirkend wie
    Dihydroergotoxin
    Dihydroergotoxin-Mesilat
    Ciclonicat
    Vinburin
    Vinpocetin
    Vincamin

38. Bronchospasmolytika, wie
    Ipratropiumbromid
    Chromoglycinsäure
    Sobrerol

39. Antiallergica, wie
    Ketotifenfumarat
    Procaterol
    Tiaramid
    Tranilast

40. Röntgenkontrastmittel, wie
    Iopanoesäureethylester

41. Hypnotika, Sedativa, wie
    Flurazepam
    Nitrazepam
    Lorazepam

42. Psychopharmaka, wie
    Oxazepam
    Diazepam
    Bromazepam

Die erfindungsgemäßen Lipidteilchen besitzen bei Raumptemperatur einen festen Aggregatzustand und weisen folgende Zusammensetzung auf:

5 —70 Gew.-% Lipid oder Lipidgemisch
0,01—70 Gew.-% grenzflächenaktive Substanzen
0,05—25 Gew.-% Wirkstoff

sowie andere Zusätze, die gegebenenfalls die Herstellung der Lipidnanopellets günstig beeinflussen, wie z.B. Peptisatoren und Suspensionsmittler.

Das Verhältnis von Lipid zu grenzflächenaktivem Stoff in den Lipidteilchen beträgt 1:0,01—1:2,2, vorzugsweise 1:0,22—1:1,2. Mischungsverhältnisse von 1:1—1:0,22 (Lipid:grenzflächenaktiven Stoff) sind ganz besonders bevorzugt.

Die Herstellung der Lipidnanopellets kann erfindungsgemäß dadurch erfolgen, daß das Lipid oder Lipidgemisch geschmolzen wird. Gleichzeitig wird die benötigte Menge destilliertes Wasser auf die gleiche Temperatur erwärmt. Die grenzflächenaktiven Stoffe werden je nach Art entweder zusammen mit dem Lipid geschmolzen, im Lipid oder im Wasser gelöst bzw. dispergiert. Die Wirkstoffe werden ebenfalls zusammen mit dem Lipid geschmolzen oder in diesem gelöst oder in diesem dispergiert. Gegebenenfalls kann das Einbringen wie angegeben über ein Lösungsmittel und Abdampfen des Lösungsmittels erfolgen. Die warme, wässrige Phase wird dem geschmolzenen Lipid zugegeben und mit ihm durchmischt und dann mit einem hochtourigen Rührer dispergiert und unter Rühren bis unterhalb des Schmelzpunktes des Lipids bzw. bis auf Zimmertemperatur abgekühlt. An die Behandlung mit dem hochtourigen Rührer schließt sich in der Regel eine Ultraschallbehandlung bei Frequenzen und Zeiträumen bis zum Erreichen der gewünschten Partikelgröße von 50 bis 1 000 nm an. Die Suspension enthält die Lipidteilchen in einer Konzentration von 1—20 Gew.-%, vorzugsweise 8—15 Gew.-%. Ganz besonders bevorzugt ist eine Konzentration von etwa 10 Gew.-%.

Es liegt eine wässrige Suspension von Lipidnanopellets vor, deren Teilchengröße in einem Bereich zwischen 50 und 1 000 nm liegt. Diese Suspension ist lagerstabil und kann direkt für die Applikation verwendet werden. Zur peroralen Anwendung wird die Suspension z.B. je nach Wirkstoffart, Wirkstoffgehalt und therapeutischer Dosis verabreicht wie sie bei flüssigen Arzneiformen üblich ist. Es ist jedoch auch möglich, die Lipidnanopellets aus der Suspension durch an sich bekannte Methoden abzutrennen oder anzureichern. Beispielsweise entsteht nach Ultrazentrifugieren und anschließender Lyophlisierung ein Pulver, das eine weitere stabile Form darstellt. Das trockene Lyophylisat kann als solches in therapeutischen Dosen abgeteilt, z.B. in Form von Pulver, Tabletten, Kapseln, verabreicht werden.

Der Vorteil der erfindungsgemäßen Lipidnanopellets liegt in der physiologischen Zusammensetzung des Arzneistoffträgersystems und der einfachen Herstellungsart. Besonders von Vorteil ist, daß aufgrund der hohen Lipophilie der erfindungsgemäßen Lipidnanopellets und deren geringen Größe von 50—1 000 nm, vorzugsweise 80—800 nm, diese im Verdauungstrakt durch die Darmwand persorbiert werden und so der intakte Lipidträger mit Wirkstoff unter Umgehung der ersten Leberpassage "first pass Effekt" in das Körpergewebe eintreten kann. Durch diesen Fettabsorptionsmechanismus ist eine gute Verteilung des Wirkstoffs im Gewebe gegeben. Die erfindungsgemäß hergestellten Lipidpartikel werden ins Fettgewebe eingelagert und ergeben somit einen Depoteffekt für den in ihnen enthaltenen Wirkstoff. Durch den enzymatischen Fettabbau wird über längere Zeit Wirkstoff freigesetzt werden, als dies bislang bei herkömmlichen oral verabreichten Arzneiformen, insbesondere Retardformen, möglich war, da die Freisetzung aus üblichen Arzneiformen durch die mittlere Verweilzeit des Arzneimittels im Verdauungstrakt auf ca. 8 Stunden beschränkt ist.

Vorteilhaft ist gleichermaßen, daß durch den erzielten Depoteffekt aus den erfindungsgemäßen Lipidnanopellets die Einnahmehäufigkeit der Arzneiform auf höchstens 1 mal pro Tag beschränkt wird, im Gegensatz zu konventionellen Arzneiformen auch Retardformen, die täglich mehrmals verabreicht werden. Damit ist eine verbesserte Therapiesicherheit gewährleistet.

Ein weiterer Vorteil der erfindungsgemäßen Lipidnanopellets ist, daß die Wirkstoffe durch die verwendeten festen Lipide besser geschützt sind. Insbesonders dann, wenn sie als Suspension verabreicht werden, gegenüber bekannten öligen Emulsionen.

Nachfolgende Beispiele sollen die Erfindung erläutern:

Beispiel 1 (ohne Wirkstoff)

3 g Tristearin werden in einen Solubilisierglas (Durchmesser 3,5 cm, Länge 20 cm) im Wasserbad bei 85°C geschmolzen. 0,3 g Gelatine werden in 54,9 g destilliertem Wasser während 15 Minuten bei Zimmertemperatur quellen gelassen und dann unter vorsichtigem Erwärmen und Rühren im Wasserbad gelöst. Diese Lösung wird weitere 15 Minuten bei 85°C konstant gehalten. 1,8 g Eilecithin werden im geschmolzenen Tristearin dispergiert. Die auf 85°C temperierte Gelatinelösung wird zur geschmolzenen Fettphase gegeben und das ganze während 10 Sekunden geschüttelt und während 1,5 Minuten bei 20.000 UpM mit einem handelsüblichen Rührwerk dispergiert. Die Abkühlung erfolgt anschließend unter leichtem Rühren bis auf Zimmertemperatur.

Es wird eine ultrafeine Suspension der Lipidteilchen erhalten, deren Partikelgröße ca. 1 000 Nanometer beträgt, wobei die Konzentration der Suspension 8,5 Gew.-% beträgt. Das Verhältnis Lipid: oberflächenaktive Substanz beträgt 1:0,7.

Beispiel 2 (ohne Wirkstoff)

3 g Tristearin werden in einem Solubiliserglas i Wasserbad bei 85°C geschmolzen In dieser Schmelze werden 1,2 g Tween® 80 sowie 2,4 g Span® 80 gegeben. 53,4 g destilliertes Wasser vmn 85°C werden zur Fettphase gegeben und dann wie in Beispiel 1 dispergiert, abgekühlt, so daß eine stabile Suspension entseht, deren Lipidteilchen eine Größe von ca. 100—350 Nanometer aufweisen.

Die Konzentration der Suspension beträgt 12,4 Gew.-%. Das Verhältnis Lipid: oberflächenaktiver Substanz beträgt 1:1,2.

Beispiel 3 (ohne Wirkstoff)

3 g Tristearin werden in einem Solubilisierglas im Wasserbad bei 85°C geschmolzen. 0,06 g Natriumcholat werden in 56,34 g destilliertem Wasser gelöst und bei 85°C temperiert. 0,6 g Phospholipon® 100-H (hydriertes Soja-Lecithin) werden in 4 ml Chloroform gelöst und zum geschmolzenen Tristearin gegeben und zweimal mit je 2 ml Chloroform nachgespült. Das Chloroform wird während 15 Minuten bei 85°C unter Schütteln entfernt. Dann wird, wie in Beispiel 1, dispergiert und abgekühlt. Die Partikelgröße der Lipidteilchen in der entstandenen Suspension beträgt ca. 1 000 Nanometer.

Die Konzentration der Suspension beträgt 6,1 Gew.-%, wobei das Verhältnis Lipid: oberflächenaktive Substanz 1:0,22 beträgt.

Beispiel 4 (ohne Wirkstoff)

2 g Tristearin werden in einem 300 ml fassenden Erlenmeyer-Kolben bei 85°C geschmolzen. 0,04 g Natriumcholat werden in 200 ml destilliertem Wasser in einem 300 ml fassenden Erlenmeyer-Kolben gelöst und auf 85°C erwärmt. 0,4 g Phospholipon® 100-H werden in 4 ml Chloroform gelöst und zum geschmolzenen Tristearin gegeben. Es wird zweimal mit je 2 ml Chloroform nachgespült. Das Chloroform wird während 15 Minuten bei 85°C entfernt. Die heiße Wasserphase wird zur Fettphase gegeben und während 10 Sekunden von Hand geschüttelt, danach erfolgt Dispergierung während 1,5 Minuten mit einem hochtourigen Rührer bei 20.000 UpM und anschließend 20 Minuten Ultraschallbehandlung bei ca 20 kHz mit einem Gerät der Type Ultrasonic. Unter leichtem Rühren mit einem Magnetrührer erfolgt danach die Abkühlung auf Zimmertemperatur. Die Teilchengröße der Suspension beträgt ca. 100 Nanometer im Durchschnitt, gemessen entlang der längsten sichtbaren Ausdehnung der Partikel. Die Konzentration der Suspension beträgt 1,2 Gew.-%, wobei das Verhältnis Lipid: oberflächenaktive Substanz 1:0,22 ist.

Beispiel 5 (ohne Wirkstoff)

6 g Tristearin werden, wie in Beispiel 4 angegeben, bei 85°C geschmolzen. 4 g Magermilch werden in 200 ml destilliertem Wasser in einem 300 ml-Erlenmeyer-Kolben dispergiert und auf 85°C temperiert. 2 g Phospholipon® 100-H werden in 5 ml Chloroform gelöst, zum geschmolzenen Tristearin gegeben und zweimal mit je 2 ml Chloroform nachgespült. Die Suspension wird durch die in Beispiel 4 angegebene Arbeitsweise hergestellt. Die Partikelgröße beträgt ca. 100—400 Nanometer.

Die Konzentration der Suspension beträgt ca. 4 Gew.-%, wobei das Verhältnis Lipid: oberflächenaktive Substanz ca. 1:0,3 ist.

Beispiel 6 (ohne Wirkstoff)

10 g Propylenglykoldistearat werden, wie in Beispiel 4 angegeben, bei 85°C geschmolzen. 0,2 g Natriumcholat werden wie in Beispiel 4 in destilliertem Wasser gelöst und auf 85°C temperiert. 2 g Phospholipon® 100-H werden in 5 ml Chloroform gelöst, zum geschmolzenen Propylenglykoldistearat gegeben und dann, wie in Beispiel 4 angegeben, die Suspension hergestellt. Die Partikelgröße beträgt ca. 100 Nanometer. Die Konzentration der Suspension beträgt ca. 5,8 Gew.-%, wobei das Verhältnis Lipid: oberflächenaktive Substanz 1:0,22 ist.

Beispiel 7 (wirkstoffhaltig)

Zusammen mit 2 g Tristearin werden 0,6 g Testosteronundecanoat im Wasserbad bei 85°C geschmolzen. Anschließend wird in gleicher Weise wie in Beispiel 4 zur Herstellung der Suspension weitergearbeitet. Die Partikelgröße der wirkstoffhaltigen Lipidteilchen beträgt ca. 50—60 Nanometer.

Die Konzentration der Suspension beträgt ca. 1,5 Gew.-%, wobei das Verhältnis Lipid: oberflächenaktive Substanz 1:0,22 ist. Der Wirkstoffanteil in den Lipidnanopellets beträgt 19,7 Gew.-%.

Beispiel 8 (wirkstoffhaltig)

2 g Octadecanol (Stearylalkohol) und 0,6 g Testosteronundecanoat wherein wie in Beispiel 4 bei 85°C geschmolzen und dann, wie in Beispiel 4 angegeben, die Suspension der Lipidteilchen hergestellt. Die Partikelgröße beträgt 100 Nanometer im Durchschnitt.

Die Konzentration der Suspension beträgt ca. 1,5 Gew.-%, wobei das Verhältnis Lipid: oberflächenaktive Substanz 1:0,22 ist.

Der Wirkstoffanteil in den Lipidnanopellets beträgt 19,7 Gew.-%.

Beispiel 9 (wirkstoffhaltig)

2,0 g Polyethylenglykolsorbitanmonooleat (Tween® 80) werden in einem Becherglas vorgelegt, 0,2 g Beclobrat zugegeben und unter Rühren und Erwärmen auf ca. 70°C gelöst. 20 g Stearylalkohol werden in einem separaten Becherglas geschmolzen. Beide Schmelzen werden unter Rühren zusammengegeben. Es entsteht eine klare Lösung. In einem 500 ml Erlenmeyer-Kolben werden 178 g Wasser vorgelegt und auf 70°C erwärmt. Die Lipidphase wird in das Wasser eingerührt, danach erfolgt Dispergierung während 5 Minuten durch Ultraschallbehandlung bei 35 kHz. Unter leichtem Rühren erfolgt Abkühlung auf Zimmertemperatur. Die entstehende Suspension wird mit weiteren 100 g Wasser verdünnt. Die entstandene Partikelgröße der Lipidteilchen beträgt ca. 800 Nanometer.

Die Konzentration der Suspension beträgt 7,4 Gew.-%. Das Verhältnis Lipid: oberfläkhenaktive Substanz beträgt 1:0,1. Der Wirkstoffanteil in den Lipidnanopellets beträgt 0,9 Gew.-%.

Beispiel 10 (wirkstoffhaltig)

2,0 g Polyethylenglykolsorbitanmonooleat (Tween® 80) werden auf 70°C erwärmt. Darin werden 0,2 g Molsidomin eingerührt. In einem separaten Gefäß werden 20 g Stearylalkohol auf 70°C erwärmt und geschmolzen. Das Lipid wird in die wirkstoffhaltige Lösung eingerührt. Es entsteht eine klare Lösung. In einem 500 ml Erlenmeyer-Kolben werden 178 g Wasser vorgelegt und auf 70°C erwärmt. Die Wirkstoffhaltige Lipidphase wird in das Wasser eingerührt und anschließend während 20 Minuten mit Ultraschall bei ca. 35 kHz behandelt. Anschließend erfolgt Abkühlung auf Zimmertemperatur. Die Teilchengröße der entstandenen Suspension beträgt ca. 500 Nanometer. Die Konzentration der Suspension beträgt 11,1 Gew.-%, wobei das Verhältnis Lipid:oberflächenaktive Substanz 1:0,1 ist.

Der Wirkstoffanteil in den Lipidnanopellets beträgt 0,9 Gew.-%.

Beispiel 11 (wirkstoffhaltig)

2,0 g Polethylenglykolsorbitanmonostearat (Tween® 60) wird in einem Becherglas auf 70°C erwärmt. 0,2 g Nifedipin werden unter Rühren darin gelöst. 20,0 g Stearylalkohol werden in einem separaten Becherglas auf 70°C erwärmt. Das lipid wird unter Rühren zu der wirkstoffhaltigen Mischung gegeben. Es entsteht eine klare Lösung. In einem 500 ml Erlenmeyer-Kolben werden 178 g Wasser vorgelegt und auf 70°C erwärmt. Die Lipidphase wird in das Wasser eingerührt und anschließend durch Ultraschall über 5 Minuten bei 35 kHz dispergiert. Unter leichtem Rühren erfolgt danach Abkühlung auf Zimmertemperatur. Die Teilchengröße der Lipidnanopellets beträgt ca. 800 Nanometer.

Die Konzentration der Suspension beträgt 11,1 Gew.-%. Das Verhältnis Lipid: oberflächenaktive Substanz ist 1:0,1.

Der Wirkstoffanteil in den Lipidnanopellets beträgt 0,9 Gew.-%.

Beispiel 12 (wirkstoffhaltig)

2,25 g Polyethylenglykolsorbitanmonooleat (Tween® 80) werden auf 70°C erwärmt. Darin werden 0,15 g Vincamin dispergiert. 22,5 Stearylalkohol werden gleichfalls auf 70°C vorgewärmt und der wirkstoffhaltigen Dispersion zugegeben. Es entsteht eine klare Lösung. In einem 500 ml Erlenmeyer-Kolben werden 178 g Wasser vorgelegt und auf 70°C erwärmt. Unter Rühren wird darin die wirkstoffhaltige Lipidphase eingetragen und durch Ultraschall während 15 Minuten bei 35 kHz dispergiert. Unter leichtem Rühren erfolgt Abkühlung auf Zimmertemperatur. Anschließend wird die Suspension mit 100 g Eiswasser versetzt. Die Teilchengröße der Suspension beträgt ca. 400 Nanometer.

Die Konzentration der Suspension beträgt 8,2 Gew.-%. Der Wirkstoffanteil in den Lipidnanopellets beträgt 0,6 Gew.-%.

Beispiel 13 (wirkstoffhaltig)

2,0 g Polyethylenglykolsorbitanmonostearat (Tween® 60) werden auf 70°C erwärmt. Darin werden 0,4 g Flurazepam dispergiert. Zu dieser Dispersion werden 20 g Stearylalkohol, die separat auf 70°C vorgewärmt wurden, gegeben. Es entsteht eine klare Lösung. In einem 500 ml Erlenmeyer-Kolben werden 178 g Wasser vorgelegt und auf 70°C erwärmt. Die wirkstoffhaltige Lipidphase wird unter Rühren in das Wasser gegeben. Anschließend erfolgt Dispergierung während 7 Minuten mit Ultraschall bei ca. 35 kHz. Unter leichtem Rühren mit einem Magnetrührer erfolgt hernach Abkühlung auf Zimmertemperatur. Die Teilchengröße der entstandenen Lipidnanopellets beträgt ca. 900 Nanometer.

Die Konzentration der Suspension ist 11,2 Gew.-%, wobei das Verhältnis Lipid: oberflächenaktive Substanz 1:0,1 beträgt.

Der Wirkstoffanteil in den Lipidnanopellets beträgt 1,8 Gew.-%.

Beispiel 14 (wirkstoffhaltig)

20,0 g Stearylalkohol werden auf 70°C erwärmt. In die Schmelze werden 2,0 g Sojabohnenlecithin und 0,6 g Indometacin eingerührt. Es entsteht eine klare Mischung. In einem 500 ml Erlenmeyer-Kolben werden 278 g Wasser auf 70°C erwärmt. Die Lipidmischung wird in das Wasser eingerührt und anschließend mit Ultraschall während 10 Minuten bei 35 kHz dispergiert. Unter leichtem Rühren erfolgt Abkühlung auf Zimmertemperatur. Die Größe der entstandenen wirkstoffhaltigen Lipidteilchen beträgt ca. 200 Nanometer.

Die Konzentration der Suspension beträgt 7,5 Gew.-%, wobei das Verhältnis Lipid: oberflächenaktive Substanz 1:0,1 ist.

Der Wirkstoffanteil in den Lipidnonopellets beträgt 2,65 Gew.-%.

Beispiel 15 (wirkstoffhaltig)

20,0 g Stearylalkohol werden bei 70°C geschmolzen. Darin wird eine Mischung aus 2,0 g Polyethylenglykolsorbitanmonooleat (Tween® 80) und 0,2 g Bromazepam gelöst. Es entsteht eine klare Lösung. In einem 500 ml Erlenmeyer-Kolben werden 178 g Wasser vorgelegt und ebenfalls auf 70°C erwärmt. Darin wird die Lipidphase eingerührt und anschließend durch Ultraschall während 10 Minuten bei 35 kHz dispergiert. Unter fortwährendem Rühren erfolgt Abkühlung auf Zimmertemperatur. Anschließend werden 100 g Eiswasser zu der entstandenen Suspension gegeben. Die Teilchengröße der entstandenen Lipidnanopellets beträgt ca. 800 Nanometer.

Die Konzentration der Suspension beträgt 7,4 Gew.-%, wobei das Verhältnis Lipid: oberflächenaktive Substanz 1:0,1 ist.

Der Wirkstoffanteil in den Lipidnanopellets beträgt 0,9 Gew.-%.

**Patentansprüche**

1. Arzneimittelhaltiges Trägersystem zur peroralen Anwendung, dadurch gekennzeichnet, daß das Trägersystem aus Lipidnanopellets die bei Raumtemperatur einen festen Aggregatzustand besitzen mit einer Teilchengröße von 50—1 000 Nanometer, insbesondere 80—800 Nanometer, in Form einer wässrigen, kolloidalen Suspension besteht, wobei die Lipidteilchen in der Suspension in einer Konzentration von 1—20 Gew.-% vorliegen, die Lipidteilen aus einem Gemisch von Lipiden mit grenzflächenaktiven Substanzen bestehen, deren Verhältnis in den Teilchen 1:0,01 bis 1:2,2, vorzugsweise 1:0,22 bis 1:1,2 insbesondere 1:1 bis 1:0,22 beträgt und die Teilchen 5—70 Gew.-% Lipide, 0,01—70 Gew.-% grenzflächenaktive Stoffe und 0,05—25 Gew.-% Wirkstoff enthalten.

2. Arzneimittelhaltiges Trägersystem nach Anspruch 1, dadurch gekennzeichnet, daß als Lipid oder Lipidgemisch gesättigte, geradkettige Fettsäuren mit 12—30 Kohlenstoffatomen wie Laurinsäure, Myristinsäure, Plamitinsäure, Stearinsäure, Arachinsäure, Behensäure, Lignocerinsäure, Cerotinsäure, Mellisinsäure, deren Mono-, Di- und Triester des Glycerins sowie anderer mehrwertiger Alkohole wie zum Beispiel Ethylenglykol, Propylenglykol, Manitol und Sorbitol verwendet werden.

3. Arzneimittelhaltiges Trägersystem nach Anspruch 1, dadurch gekennzeichnet, daß als Lipid oder Lipidgemisch Fettalohole mit 12—22 Kohlenstoffatomen wie Laurylakohol, Myristylalkohol, Cetylalkohol, Stearylalkohol, Arachidylalkohol, Behenylalkohol werdendet werden.

4. Arzneimittelhaltiges Trägersystem nach Anspruch 1, dadurch gekennzeichnet, daß als Lipid oder Lipidgemisch Wachsalkohole mit 24—30 Kohlenstoffatomen wie Lignocerylalkohol, Cerylalkohol, Cerotylalkohol oder Myricylalkohol verwendet werden.

5. Arzneimittelhaltiges Trägersystem nach Anspruch 1, dadurch gekennzeichnet, daß das Lipid als Gemisch aus den Lipiden gemäß Anspruch 2, 3 und 4 vorliegt.

6. Arzneimittelhaltiges Trägersystem nach Anspruch 1 dadurch gekennzeichnet, daß als grenzflächenaktiver Stoff oder Stoffgemisch natürliche Gallensalze wie Natriumcholat, Natriumdehydrocholat, Natriumdeoxycholat, Natriumglykocholat, Natriumaurocholat verwendet werden.

7. Arzneimittelhaltiges Trägersystem nach Anspruch 1, dadurch gekennzeichnet, daß als grenzflächenaktive Substanzen tierische oder pflanzliche Phospholipide wie Lecithine und ihre hydrierten Formen sowie Polypeptide wie Gelatine mit ihren modifizierten Formen verwendet werden.

8. Arzneimittelhaltiges Trägersystem nach Anspruch 1, dadurch gekennzeichnet, daß als synthetische grenzflächenaktive Substanzen die Salze der Sulfobernsteinsäureester, Polyoxyethylensorbitanester, Sorbitanester und Sorbitanether, Polyoxyethylenfettalkoholether, Polyoxyethylenstearinsäureester, sowie Mischkondensate von Polyoxyethylen- mit Polyoxypropylenether, ethoxylierte gesättigte Glyceride, partielle Fettsäure-Glyceride und Polyglycide verwendet werden.

9. Arzneimittelhaltiges Trägersystem nach Anspruch 1, dadurch gekennzeichnet, daß als grenzflächenaktive Substanzen ein Gemisch aus den grenzflächenaktiven Substanzen der Ansprüche 6, 7 und 8 verwendet wird.

10. Arzneimittelhaltiges Trägersystem nach Anspruch 1, dadurch gekennzeichnet, daß die Lipidteilchen in der wässrigen Suspension in einer Konzentration von 8—15 Gew.-%, insbesondere 10 Gew.-% vorliegen.

11. Arzneimittelhaltiges Trägersystem nach Anspruch 1, dadurch gekennzeichnet, daß der Wirkstoffanteil oberhalb des Schmelzpunktes des Gemisches aus Lipid und grenzflächenaktiver Substanz in gelöster oder geschmolzener Form vorliegt.

12. Arzneimittelhaltiges Trägersystem nach Anspruch 1, dadurch gekennzeichnet, daß der Wirkstoff in den Lipidnanopellets nach Erkalten auf Raumtemperatur bzw. unterhalb des Schmelzpunktes des Gemisches gelöst oder kristallin oder amorph oder als Gemisch aus solchen kristallographischen Zuständen vorliegt.

13. Arzneimittelhaltiges Trägersystem nach Anspruch 1, dadurch gekennzeichnet, daß der Wirkstoff auf Lipidsenkern, Vasodilatoren, Calciumantagonisten, Steroidhormonen, Antirheumatica, Hypnotika und Psychopharmaka ausgewält ist.

**Revendications**

1. Système de support contenant des substances médicamenteuses pour administration par voie orale, caractérisé en ce que le système de support est constitué d'une suspension aqueuse colloïdale formée de nanogranulés de lipides qui se présentent sous forme d'agrégats solides à température ambiante avec une granulométrie de 50 à 100 nanomètres et, en particulier, de 80 à 800 nanomètres, tandis que les particules de lipides sont présentes dans la suspension avec une concentration de 1 à 20% en poids, que les particules de liquides sont constituées d'un mélange de lipides et de substances tensioactives dont la proportion dans les particules est comprise dans le domaine allant de 1:0,01 à 1:2,2 et, de préférence, de 1:0,22 à 1:1,2 et, plus particulièrement encore, de 1:1 à 1:0,22, et que les particulés contiennent 5 à 70% en poids de lipides, 0,01 à 70% en poids de substances tensioactives et 0,05 à 25% en poids de substances actives.

2. Système de support contenant des substances médicamenteuses selon la revendication 1, caractérisé en ce que l'on utilise comme lipides ou comme mélanges de lipides des acides gras saturés à chaîne droite à 12 à 30 atomes de carbone tels que l'acide laurique, l'acide myristique, l'acide palmitique, l'acide stéarique, l'acide arachique, l'acide béhénique, l'acide lignosérique, l'acide cérotique, l'acide mélissique, leurs mono- di- et triesters de la glycérine ainsi que d'autres alcools polyvalents comme par exemple l'éthylèneglycol, le propylèneglycol, le manitol et le sorbitol.

3. Système de support contenant des substances médicamenteuses selon la revendication 1, caractérisé en ce que l'on utilise comme lipides ou comme mélange de lipides des alcools gras à 12 à 22 atomes de carbone tels que l'alcool laurylique, l'alcool myristylique, l'alcool cétylique, l'alcool stéarylique, l'alcool arachidylique, l'alcool béhénique.

4. Système de support contenant des substances médicamenteuses selon la revendication 1, caractérisé en ce que l'on utise comme lipides ou comme mélange de lipides des alcools de cire à 24 à 30 atomes de carbone tels que l'alcool lignocérylique, l'alcool cérylique, l'alcool cérotylique ou l'alcool myricylique.

5. Système de support contenant des substances médicamenteuses selon la revendication 1, caractérisé en ce que le lipide se présente sous forme d'un mélange des lipides selon les revendications 2, 3 et 4.

6. Système de support contenant des substances médicamenteuses selon la revendication 1, caractérisé en ce que l'on utilise comme substances tensioactives ou comme mélange de telles substances des sels biliaires naturels tels que le cholate de sodium, le déhydrocholate de sodium, le désoxycholate de sodium, le glycocholate de sodium, le laurocholate de sodium.

7. Système de support contenant les substances médicamenteuses selon la revendication 1, caractérisé ef ce que l'on utilise comme substances tensioactives des phospholipides animaux ou végétaux tels que les lécithines et leurs formes hydrogénées ainsi que des polypeptides tels que les gélatines avec leurs formes modifiées.

8. Système de support contenant les substances médicamenteuses selon la revendication 1, caractérisé en ce que l'on utilise comme substances synthétiques tensioactives les sels des esters de l'acide sulfosuccinique, des esters de polyoxyéthylènesorbitane, des esters de sorbitane et des éthers de sorbitane, des éthers d'alcools gras de polyoxyéthylène, des stéarates de polyoxyéthylène ainsi que des condensats mixtes d'éthers de polyoxyéthylène et d'éthers de polyoxypropylène, des glycérides saturés éthoxylés, des glycérides d'acides gras partiels et des polyglycides.

9. Système de support contenant des substances médicamenteuses selon la revendication 1, caractérisé en ce que l'on utilise comme substances tensioactives un mélange des substances tensioactives des revendications 6, 7 et 8.

10. Système de support contenant des substances médicamenteuses selon la revendication 1, caractérisé en ce que les particules de lipides se présentent dans la suspension aqueuse avec une concentration de 8 à 15% en poids et, plus particulièrement, de 10% en poids.

11. Système de support contenant des substances médicamenteuses selon la revendication 1, caractérisé en ce que la proportion de substance active au-dessus du point de fusion du mélange de lipides et de substances tensioactives se présente à l'état dissous ou fondu.

12. Système de support contenant des substances médicamenteuses selon la revendication 1, caractérisé en ce que la substance active se présente dans les monogranulés de lipides après refroidissement à température ambiante en dessous du point de fusion du mélange à l'état dissous ou cristallin ou amorphe ou sous forme d'un mélange de ces états cristallographiques.

13. Système de support contenant des substances médicamenteuses selon la revendication 1, caractérisé en ce que la substance active est choisie parmi les réducteurs de lipides, les vasodilatateurs, les antagonistes du calcium, les hormones stéroïdales, les antirhumatismaux, les hypnotiques et les produits psychopharmaceutiques.

**Claims**

1. Medicament-containing carrier system for peroral use, characterized in that the carrier system comprises lipid nanopellets, which have a solid aggregate state at room temperature, having a particle size of 50—1,000 nanometers, in particular 80—800 nanometers, in the form of an aqueous colloidal suspension, the lipid particles being present in the suspension in a concentration of 1—20% by weight, the lipid particles comprising a mixture of lipids with boundary surface-active substances, whose ratio in the particles is 1:0.01 to 1:2.2, preferably 1:0.22 to 1:1.2, in particular 1:1 to 1:0.22, and the particles containing 5—70% by weight of lipids, 0.01—70% by weight of boundary surface-active substances and 0.05—25% by weight of active compound.

2. Medicament-containing carrier system according to Claim 1, characterized in that saturated, straight-chain fatty acids having 12—30 carbon atoms such as lauric acid, myristic acid, palmitic acid, stearic acid, arachic acid, behenic acid, lignoceric acid, cerotic acid, melissic acid, ther glycerol mono-, di- and triesters and other polyhydric alcohols such as, for example, ethylene glycol, propylene glycol, mannitol and sorbitol are used as the lipid or lipid mixture.

3. Medicament-containing carrier system according to Claim 1, characterized in that fatty alcohols having 12—22 carbon atoms such as lauryl alcohol, myristyl alcohol, cetyl alcohol, stearyl alcohol, arachidyl alcohol and behenyl alcohol are used as the lipid or lipid mixture.

4. Medicament-containing carrier system according to Claim 1, characterized in that wax alcohols having 24—30 carbon atoms such as lignoceryl alcohol, ceryl alcohol, cerotyl alcohol or myricyl alcohol are used as the lipid or lipid mixture.

5. Medicament-containing carrier system according to Claim 1, characterized in that the lipid is present as a mixture of the lipids according to Claims 2, 3 and 4.

6. Medicament-containing carrier system according to Claim 1, characterized in that natural bile salts such as sodium cholate, sodium dehydrocholate, sodium deoxycholate, sodium glycoholate and sodium laurocholate are used as the boundary surface-active substance or substance mixture.

7. Medicament-containing carrier system according to Claim 1, characterized in that animal or vegetable phospholipids such as lecithins and their hydrogenated forms and also polypeptides such as gelatine and its modified forms are used as the boundary surface-active substances.

8. Medicament-containing carrier system according to Claim 1, characterized in that the salts of sulphosuccinic acid esters, polyoxyethylene sorbitan esters, sorbitan esters and sorbitan ethers, polyoxyethylene fatty alcohol ethers, polyoxyethylene stearic acid esters, and mixed condensates of polyoxyethylene ethers with polyoxypropylene ethers, ethoxylated saturated glycerides, partial fatty acid glycerides and polyglycides are used as synthetic boundary surface-active substances.

9. Medicament-containing carrier system according to Claim 1, characterized in that a mixture of the boundary surface-active substances of Claims 6, 7 and 8 is used as boundary surface-active substance.

10. Medicament-containing carrier system according to Claim 1, characterized in that the lipid particles are present in the aqueous suspension in a concentration of 8—15% by weight, in particular 10% by weight.

11. Medicament-containing carrier system according to Claim 1, characterized in that the active compound fraction is present in dissolved or molten form above the melting point of the mixture of lipid and boundary surface-active substance.

12. Medicament-containing carrier system according to Claim 1, characterized in that the active compound is present in the lipid nanopellets after cooling to room temperature or below the melting point of the mixture in dissolved, crystalline or amorphous form or as a mixture of such crystallographic states.

13. Medicament-containing carrier system according to Claim 1, characterized in that the active compound is selected from hypolipidaemics, vasodilators, calcium antagonists, steroid hormones, antirheumatics, hypnotics and psychopharmceuticals.